Europäisches Patentamt

European Patent Office

Office européen des brevets

(19)

(11) Numéro de publication: **0 199 634**

**A1**

(12) **DEMANDE DE BREVET EUROPEEN**

(21) Numéro de dépôt: **86400770.3**

(22) Date de dépôt: **10.04.86**

(51) Int. Cl.4: **C12M 1/00**

(30) Priorité: **16.04.85 FR 8505718**

(43) Date de publication de la demande:
**29.10.86 Bulletin  86/44**

(84) Etats contractants désignés:
**BE DE GB IT NL**

(71) Demandeur: **SOCIETE GENERALE POUR LES TECHNIQUES NOUVELLES S.G.N. Société anonyme dite:**
**1, rue des Hérons Montigny-le-Bretonneux**
**F-78184 Saint-Quentin en Yvelines**
**Cedex(FR)**

(72) Inventeur: **Henry, Michel**
**19, Rue F. David**
**F-78100 St. Germain-en-Laye(FR)**
Inventeur: **Leulliette, Laurent**
**2, Rue de Limoges**
**F-93800 Epinay-Sur-Seine(FR)**

(74) Mandataire: **Combe, André et al**
**CABINET BEAU DE LOMENIE 55 rue d'Amsterdam**
**F-75008 Paris(FR)**

(54) **Procédé et dispositif de production d'éthanol par fermentation alcoolique.**

(57) La première invention concerne un dispositif pour la fermentation alcoolique de liquides sucrés au moyen de levures floculées, caractérisé en ce qu'il se compose d'un fermenteur à deux étages : un étage de fermentation (4) et un étage de décantation (5) situé au-dessus de l'étage de fermentation, que chaque étage est muni d'un garnissage (6,9)-,qu'entre les étages est placé un déflecteur (13) et que l'étage de décantation a un diamètre égal ou supérieur à l'étage de fermentation, elle concerne également un procédé de fermentation au moyen de levures floculées, le procédé utilisant le dispositif décrit ci-dessus.

Fig.1

EP 0 199 634 A1

# Procédé et dispositif de production d'éthanol par fermentation alcoolique.

Il est connu depuis bien longtemps que les levures transforment le sucre en alcool, la réaction est dite de fermentation alcoolique. Cette réaction est couramment utilisée non seulement pour produire des boissons alcoolisées mais aussi pour obtenir des alcools purs après distillation du liquide fermenté.

On procède habituellement à cette opération dans les installations de distillerie avec un fermenteur du type infiniment mélangé. Après ensemencement du fermenteur par des levures Saccharomyces cerevisiae qui peuvent être soit des souches particulières, soit plus simplement de la levure de boulanger, le substrat sucré est traité par acidification puis addition de nutriments avant de passer dans le fermenteur. Là, les levures à l'état de cellules libres constamment agitées transforment le sucre en éthanol et en gaz carbonique. Le temps de séjour du substrat dans le fermenteur est de l'ordre de 20 h.

Ce temps de séjour est relativement long. Un procédé décrit dans le brevet français n° 2 416 263 permet d'abaisser les temps de fermentation à environ 3 h. Pour cela, il est procédé à une extraction continue de l'éthanol produit de façon à maintenir sa concentration dans le fermenteur à une valeur en dessous de 5% en volume, car l'éthanol commence à inhiber la réaction de fermentation au-dessus de ce seuil.

Un autre inconvénient des procédés connus de ce type est de produire des levures, ces levures pour se nourrir et se reproduire consomment une certaine quantité de substrat qui n'est donc pas valorisée enalcool. Par ailleurs, ce phénomène nécessite de l'exploitant qu'il procède à une extraction continuelle des levures par centrifugation sur le liquide fermenté sortant fortement chargé en levures, cela de façon à ne pas engorger le fermenteur par un surplus de cellules dont la consommation en substrat augmenterait trop et de façon à ne pas "encrasser" les colonnes utilisées pour l'extraction ultérieure de l'éthanol.

Les levures utilisées dans ces procédés industriels sont sous forme de cellules libres. Elles sont introduites sous cette forme et y demeurent.

Selon les brevets US 4 413 058 et UK 2 065 699 des micro-organismes floculés peuvent avantageusement être utilisés pour la production d'éthanol.

Dans le brevet UK 2 065 699 par exemple, est décrite une installation comprenant un fermenteur de type infiniment mélangé continuellement agité sur tout son volume et une cuve de floculation extérieure au fermenteur. Les micro-organismes sont introduits sous forme de cellules libres dans la cuve et ils floculent dans ladite cuve. Les micro-organismes floculés sont ensuite injectés dans le fermenteur où ils transforment le sucre en éthanol. Le liquide alcoolisé sortant du fermenteur contient une quantité importante de micro-organismes et nécessite leur séparation du liquide par centrifugation.

L'inconvénient de tels procédés réside dans le recours à la centrifugation. Outre le fait que l'entretien des centrifugeuses est onéreux et pose des problèmes de disponibilité sur l'installation, la séparation à grande vitesse "casse" les micro-organismes floculés et les remet sous forme libre, de sorte qu'il est nécessaire de les faire floculer à nouveau avant de les réutiliser en fermentation.

Au contraire des procédés connus, la demanderesse propose un procédé continu de fermentation alcoolique de substrats sucrés utilisant des levures floculées mais ne nécessitant pas de centrifugation pour les séparer. Ce procédé présente donc entre autres avantages celui de conserver la plus grande partie des levures floculées sous cette forme. De plus, dans ce procédé le milieu de fermentation n'est pas infiniment mélangé, c'est-à-dire que la concentration en alcool et en levures n'est pas identique en tout point du volume de fermenteur.

En effet, selon le procédé développé, dans le bas de l'étage de fermentation où a lieu la réaction de fermentation, le substrat s'écoulant du bas vers le haut se trouve la majorité des levures floculées 90-95% du total des levures) tandis que dans le haut dudit étage de fermentation 5-10% des levures se retrouvent.

Pour mettre en oeuvre ce procédé, la demanderesse utilise le fermenteur objet de l'invention, caractérisé en ce qu'il comporte deux étages : un étage inférieur dit de fermentation et un étage supérieur dit de décantation, chacun desdits étages est muni d'un garnissage occupant entre 20% et 50% du volume total de l'étage correspondant, l'étage de décantation étant muni de plus d'un déflecteur.

Ainsi l'invention concerne pour l'essentiel un

Dispositif pour la fermentation alcoolique de liquides sucrés au moyen de levures floculées comportant un étage de fermentation constitué d'un fond et d'un corps de forme cylindrique à axe vertical et un étage de décantation situé au-dessus de l'étage de fermentation constitué d'un corps cylindrique à axe vertical relié tronconiquement au corps cylindrique de l'étage de fermentation et un couvercle fermant ledit étage de décantation, ledit dispositif comportant une tubulure d'amenée des liquides sucrés dans le bas de l'étage de fermentation, une tubulure de départ de jus alcoolisés situé au haut de l'étage de décantation et une tubulure d'évacuation des gaz, ledit dispositif étant caractérisé en ce que :

-chacun des étages de fermentation (1) et de décantation (2) est constitué par une partie inférieure vide (4,5) et une partie supérieure munie d'un garnissage (6,9) maintenu entre deux grilles,

-le diamètre de l'étage de décantation est de 1 à 1,5 fois le diamètre de l'étage de fermentation de façon à ce que l'on puisse réaliser une vitesse ascensionnelle de l'ordre de 0,2 à 0,7 m/h dans l'étage de fermentation et de 0,05 à 0,1 m/h dans l'étage de décantation,

-entre les étages de fermentation et de décantation on aménage au moins un déflecteur (13),

-une tubulure (20) partant du fond de l'étage de fermentation amène le liquide contenant des levures floculées dans une cuve (21) où ledit liquide est additionné d'une quantité convenable d'acide sulfurique pour provoquer la défloculation au moins partielle desdites levures et leur régénération, une tubulure (23) comportant une pompe ramenant les levures ainsi régénérées dans l'étage de fermentation.

L'invention converne également un procédé utilisant de façon optimale ledit dispositif ; ledit procédé est caractérisé essentiellement par le fait que l'on utilise comme levures des Saccharomyces Cerevisiae et que :

-le substrat arrivant dans la partie basse, vide, de l'étage de fermentation simultanément avec de l'air est ensemencé et soumis à un mouvement ascendant,

-les flocs de levure se développent essentiellement dans ladite partie basse et vide de l'étage de fermentation et pour cela la vitesse ascensionnelle du milieu est maintenue entre 0,2 et 0,7 m/h,

-dans l'étage de decantation on assure au milieu une vitesse ascensionnelle comprise entre 0,05 et 0,1 m/h.

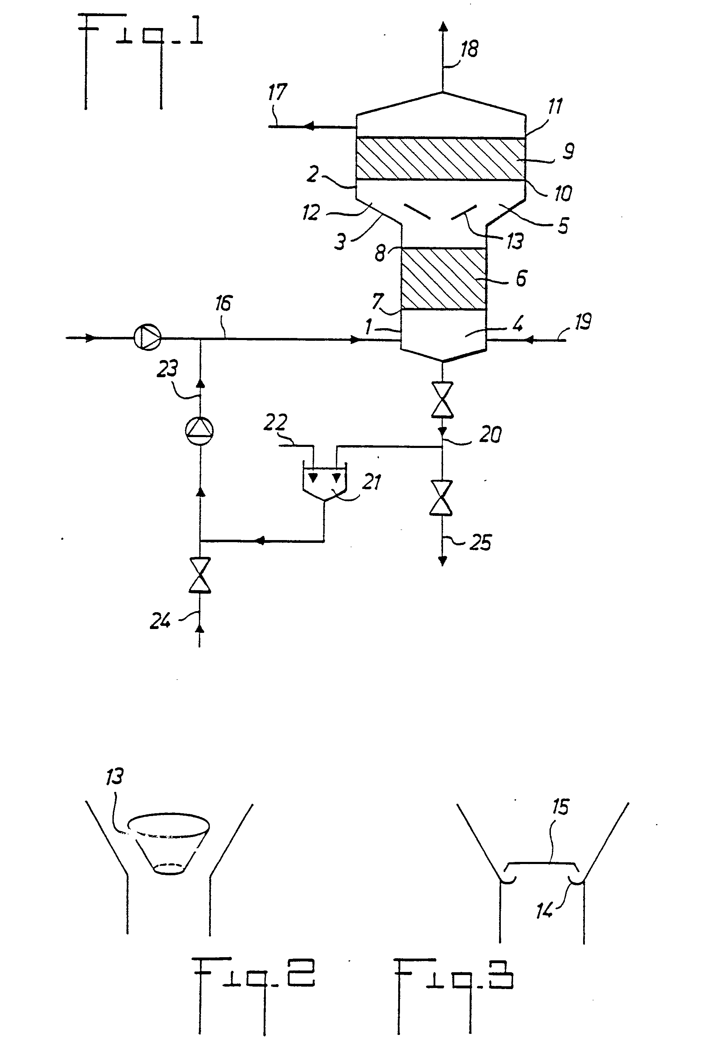

L'invention sera mieux comprise en se référant de suite à la figure 1 qui représente le fermenteur objet de l'invention.

Le fermenteur est formé de deux viroles 1 et 2, par exemple en acier, de diamètre différent, reliées entre elles par une paroi conique 3.

Le diamètre de la virole 2 supérieure est égal ou supérieur à celui de la virole 1 et de préférence il est compris entre 1 et 3,5 fois celui de la virole 1, ceci pour respecter les vitesses ascensionnelles du substrat imposées par le procédé.

Le fermenteur comprend deux étages : un étage de fermentation et un étage de décantation. L'étage de fermentation 4 correspond à la virole 1 et l'étage de décantation 5 à la partie conique 3 plus à la virole 2.

Chaque étage est muni d'un garnissage maintenu fixe entre deux grilles. Ainsi, l'étage de fermentation contient le garnissage 6 maintenu entre les grilles 7 et 8 et l'étage de décantation le garnissage 9 maintenu entre les grilles 10 et 11.

Chacun des garnissages occupe un volume égal de 20 à 50 % du volume de l'étage correspondant et est placé dans les 2/3 supérieurs de chaque étage ; le garnissage 6 sera de préférence placé dans la moitié supérieure de l'étage de fermentation, de façon à augmenter le volume de la zone 1 où a lieu la presque totalité de la réaction.

Des éléments pour le garnissage sous forme de plaquette, anneaux, billes, copeaux... en matière organique (plastique par exemple) ou minérale conviennent, un garnissage avec des anneaux ondulés en polychlorure de vinyle du type "FLOCOR R" pourra par exemple être utilisé.

En bas de l'étage de décantation, un dispositif complémentaire est installé qui permet de transformer l'écoulement ascendant unidirectionnel du substrat en un écoulement multidirectionnel plus complexe avec une fraction du substrat s'écoulant momentanément vers le bas, cela de façon à créer une légère agitation du milieu , que les particules plus lourdes (les levures floculées) commencent à décanter et à favoriser le dégazage.

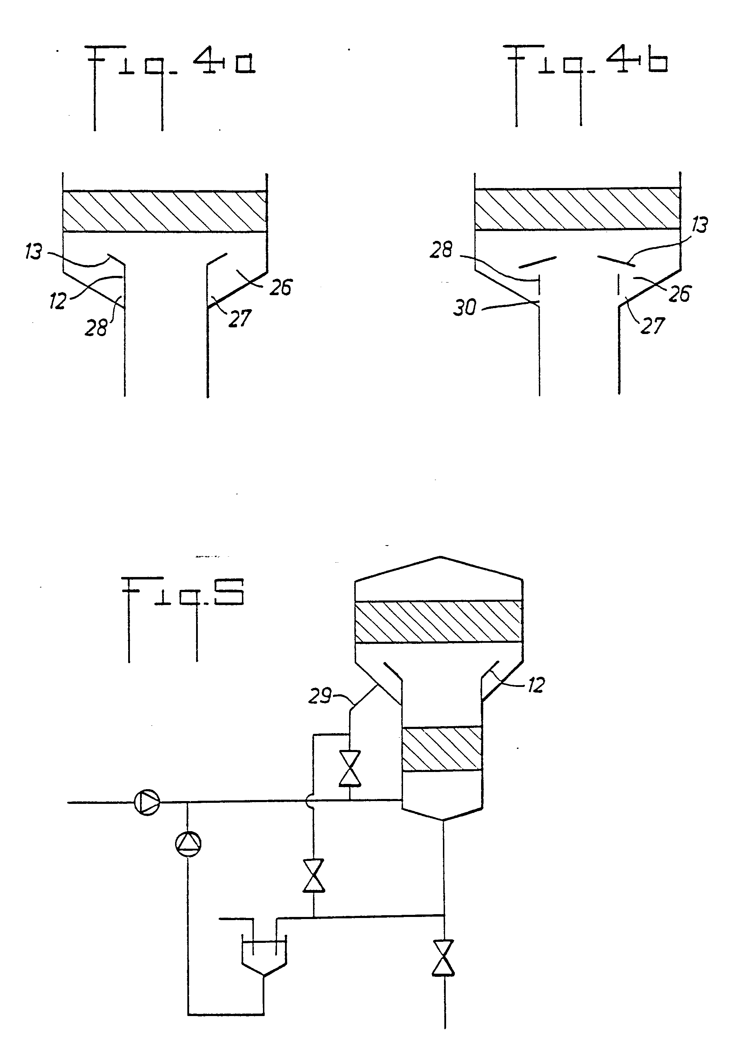

Le dispositif utilisé est connu, il s'agit de dispositifs de type déflecteur représenté sur les figures 2, 3 et 4.

Sur la figure 2, le déflecteur 12 se compose d'une paroi 13 en forme de couronne conique. De préférence, ce déflecteur est placé comme le montre la figure 2, à la limite des deux étages c'est-à-dire à l'endroit où le diamètre augmente.

Un autre type de déflecteur connu représenté figure 3 peut tout aussi bien être retenu. Il comprend une couronne 14 soudée en haut de la virole 1 et une calote 15 coiffant cette couronne.

La paroi 13 ou bien la calotte 15 sont fixées à la virole du fermenteur au moyen d'entretoises par exemple nom figurées sur les schémas.

Selon la figure 4, le déflecteur 12 représenté figures 1 et 2, est monté de façon différente. Au lieu d'être fixé directement aux parois du fermenteur, la virole 1 est prolongée dans l'étage de décantation, sur une longueur de l'ordre du diamètre de ladite virole, et la paroi conique 13 du déflecteur est fixée sur ledit prolongement 28. Sur la figure 4a, elle est soudée, sur la figure 4b des entretoises tiennent lieu de moyens de fixation.

Dans ce mode de réalisation, il est créé une zone calme 26 située entre le prolongement 28 de la virole 1 et la paroi du fermenteur. Dans cette zone, les levures décantent et sont rassemblées dans le fond 27.

Pour renvoyer les levures dans l'étage de fermentation, deux possibilités s'offrent à l'exploitant.

Selon la première, figure 5, les levures sont soutirées par une tubulure 29 débouchant sur la partie 27, puis sont éventuellement régénérées avant d'être injectées dans l'étage de fermentation par la canalisation 16.

Selon la seconde possibilité, figure 4b, dans la paroi du prolongement 28 de la virole 1 sont aménagées des ouvertures 30 de façon que le liquide chargé de levures retombe dans l'étage de fermentation.

Le substrat sucré à traiter entre par une tubulure 16 située en bas du fermenteur et en ressort par la tubulure 17 placée dans la partie haute du fermenteur et à proximité de la grille 11.

Il traverse ainsi 4 zones dans le fermenteur :

. la zone 1 située en-dessous du garnissage dans laquelle la presque totalité de la réaction de fermentation a lieu car là se trouvent 90-95 % des levures,

. la zone 2 située entre le garnissage 6 et le déflecteur, correspondant ainsi à la partie de l'étage de fermentation située audessus du garnissage 6. Dans cette zone, la réaction de fermentation se finit.

. la zone 3 située entre l'étage de fermenation et le garnissage 9, elle comprend le déflecteur 12. Dans cette zone, l'écoulement du substrat est modifié dans son orientation et sa vitesse, les levures floculées entraînées décantent,

. la zone 4 située au-dessus du garnissage 9 de laquelle le substrat "propre" est extrait et dans laquelle sont collectés les gaz formés.

Au sommet du dôme conique du fermenteur, la tubulure 18 permet l'évacuation des gaz formés qui traversent un condenseur de vapeurs avant rejet à l'atmosphère.

Il est également prévu une tubulure 19 pour l'insufflation d'air ou d'oxygène dans le bas du fermenteur.

Le fermenteur comporte de plus un dispositif permettant le soutirage des levures dans l'étage de fermentation et leur recyclage après régénération.

Ce dispositif se compose d'une tubulure 20 située dans le fond de la virole 1, par laquelle sont soutirées les levures, d'une cuve 21 contenant de l'acide sulfurique dilué amené par la canalisation 22 et d'une conduite 23 par laquelle les levures sont extraites de la cuve 21 et amenées à la canalisation 16 d'alimentation en substrat.

Outre le recyclage des levures, il est aussi possible d'introduire des levures nouvelles, soit sous forme libre, soit sous forme floculée par la canalisation 24 branchée sur la canalisation 23.

En outre, une conduite 25 branchée sur la canalisation 20 permet d'évacuer des levures inutilisables, mortes par exemple, elle a un rôle de conduite de purge.

Le milieu de réaction devant être maintenu entre 29°C et 35°C, le fermenteur est équipé d'une double enveloppe au moins sur l'étage de fermentation dans laquelle circule de l'eau chaude. Un autre moyen est de réchauffer le substrat avant introduction dans le fermenteur.

Le procédé décrit dans l'invention repose sur la mise en oeuvre du fermenteur de la figure 1.

Les substrats sucrés traités peuvent être des jus de betteraves par exemple, des sirops ou des mélasses ou encore des mélanges.

Nous précisons que le terme "mélasse" désigne une matière sucrée issue de la dernière étape de cristallisation du sucre, cette matière contenant environ 50 % en poids de sucre non extractible et des ions minéraux. Le terme "sirop" désigne le liquide issu de la concentration des jus sucrés de départ après que ceux-ci ont subi une élimination des mucilages (solides résiduels de la betterave) et un échange ionique.

Avant d'être envoyé au fermenteur, le liquide à traiter est amené à un pH inférieur à 6, de préférence compris entre 3 et 4 au moyen d'acide sulfurique dilué, ce qui permet d'éviter une possible infection bactérienne. Comme cela est le cas dans les procédés classiques, peuvent être ajoutés des nutriments tels que la magnésie (jusqu'à 0,5 g/l), le phosphate ou l'hydrogénophosphate d'ammonium (jusqu'à 2 g/l), le chlorure de calcium - (jusqu'à 0,3 g/l) ou le sulfate d'ammonium (jusqu'à 2 g/l). La concentration en saccharose du substrat qui alimente le fermenteur est de l'ordre de 80-200 g/l, un substrat moins concentré en saccharose peut évidemment être fermenté, mais l'intérêt industriel est alors moindre.

Précisons tout d'abord que les opérations d'ensemencement et de fermentation se déroulent entre 29°C et 35°C, l'opération de décantation n'est pas assujettie à ce domaine de température.

Avant toute opération de fermentation, le fermenteur est ensemencé par une solution de levures.

Deux possibilités s'offrent à l'exploitation.

Dans un premier cas, le fermenteur est rempli de levures floculées, c'est-à-dire que la floculation a lieu dans un réacteur situé hors du fermenteur, ledit réacteur étant raccordé au fermenteur par la canalisation 24.

Dans un second cas, et avantageusement, les levures sont injectées dans le fermenteur sous forme de cellules libres et elles sont amenées à floculations dans le fermenteur.

Dans les deux cas, des levures floculantes sont utilisées, c'est-à-dire des levures susceptibles de floculer, ce peuvent être par exemple des levures Saccharomyces cerevisiae.

En utilisant des levures de ce type, il est procédé de la façon suivante.

Dans une première étape d'ensemencement, une solution appelée "pied de cuve" de levures Saccharomyces cerevisiae sous forme de cellules libres est préparée par dispersion des cellules dans une solution de substrat dilué si nécessaire contenant éventuellement des nutriments, de façon que la concentration en saccharose soit comprise entre 80 et 120 g/l et la concentration en levures entre 15 et 30 g/l.

Ce "pied de cuve" est amené au fermenteur par la canalisation 16.

Dans une seconde étape de croissance et floculation des levures, le substrat sucré enrichi éventuellement en nutriments et de concentration en saccharose comprise entre 80 et 120 g/l est injecté par la canalisation 16. Le débit est réglé de façon que la vitesse ascensionnelle du substrat dans l'étage de fermentation soit de l'ordre de 0,1 à 0,15 m/h. Dans le même temps, l'oxygène nécessaire à la croissance des levures et à leur survie est diffusé au moyen de bouches en verre fritté fixées sur la canalisation 19 en bas de l'étage de fermentation. Mais trop d'oxygène entraînerait un développement excessif des levures, si bien qu'il deviendrait nécessaire de les soutirer périodiquement. Donc, on a choisi un débit d'air insufflé compris entre 2 et 4 volumes d'air par volume de liquide en fermentation et par heure.

Dans ces conditions, les levures sont floculées au bout de 12 à 60 h. Les levures floculées présentes dans l'étage de fermentation sont en fait confinées en grande partie dans la zone 1, qui contient de 90 à 95 % des levures tandis que la zone 2 n'en contient que près de 5 à 10 %.

Le garnissage 6 joue aussi un rôle important dans la floculation des cellules libres de levures : en les confinant dans la zone 1, il permet d'augmenter leur concentration de façon régulière dans cette zone jusqu'à provoquer la floculation.

Après obtention des levures floculées, la mise en régime du fermenteur est effectuée en augmentant le débit en substrat sucré ainsi que la concentration en saccharose du substrat (cela signifie que la dilution du substrat est réduite voire annulée). Le débit est augmenté jusqu'à ce que la vitesse ascensionnelle du substrat dans l'étage de fermentation atteigne 0,2 à 0,7 m/l, et la concentration en saccharose peut s'élever jusqu'à 200 g/l de substrat mais il sera préféré 140-170 g/l.

Le substrat subit la plus grande partie de sa transformation en zone 1 ; en traversant le garnissage 6, il se libère d'une partie importante des levures floculées, la réaction de fermentation se termine en haut de l'étage de fermentation en zone 2.

En régime, la concentration en levures dans l'étage de fermentation est établie entre 20 et 100 g/l d'étage de fermentation, et de préférence 80 g/l.

Ces levures ne sont pas réparties uniformément dans ledit étage : 90-95 % (en poids sec) des levures se trouvent en zone 1 et 5-10 % en zone 2.

Ce liquide alcoolisé contient encore quelques levures ; ce sont soit des levures mortes, soit quelques levures floculées entraînées par le substrat ou le dégagement gazeux, soit des cellules libres non floculées.

Le liquide passe alors dans l'étage de décantation par le déflecteur 12, puis dans le garnissage 9. Le déflecteur 12 modifie le sens de l'écoulement du liquide, de sorte que les particules les plus lourdes (levures floculées) décantent et ainsi le confinement des levures floculées dans l'étage de fermentation est assuré.

Puis le liquide alcoolisé traverse le garnissage de bas en haut. Les anneaux du garnissage créent de multiples "chicanes" dans lesquelles passe le liquide chargé en levures et en gaz dissous.

Pendant sa traversée, le liquide se dégaze et se libère en grande partie des levures restantes trop encombrantes pour passer à travers les "chicanes". De sorte que, en haut de l'étage de décantation sort par la canalisation 17 un liquide suffisamment "propre" pour être traité immédiatement dans les colonnes d'extraction de l'alcool. Le garnissage a joué un rôle de filtre. Cet effet est renforcé par le déplacement lent du substrat dans cet étage (0,05 à 0,2 m/h).

Les gaz formés sont évacués par la canalisation 18 en haut de cet étage de décantation.

Un avantage résultant de ces conditions opératoires est qu'il n'est pas nécessaire de recycler le substrat pour qu'il subisse à nouveau la fermentation alcoolique. Ainsi, au bout d'un temps de séjour de 9-10 h au plus du substrat dans le fermenteur, il ressort par la canalisation 17 un liquide contenant au moins 7 % d'alcool (% volume-volume) et en général 8,5 à 9 % en volume.

Il est également possible de régénérer et de recycler une partie des levures de façon à maintenir l'activité fermentaire. Pour cela, une fraction du milieu est soutirée par la canalisation 20 et est envoyée à la cuve 21 de régénération ; là les levures sont agitées dans un milieu acide sulfurique dilué à pH ; 3-4.

Ainsi régénérées, les levures sont renvoyées directement au fermenteur par la canalisation 23. Cette opération est un simple lavage des cellules : leur paroi est nettoyée, débarrassée des impuretés pouvant s'y trouver, les cellules retrouvent ainsi une certaine "jeunesse" et leur activité est retrouvée.

Il peut être utilisé en cours de fermentation de réintroduire des cellules libres ou floculées pour augmenter la masse des levures actives, dans ce cas elles sont injectées par la canalisation 24 et zone 1 du fermenteur.

La demanderesse présente donc un procédé et un dispositif pour la transformation en continu du saccharose en éthanol par fermentation avec des levures floculées, la floculation pouvant avantageusement être mise en oeuvre dans le dispositif objet de l'invention.

Le procédé a lieu en deux étapes principales, l'une de fermentation, l'autre de décantation, la fermentation ayant lieu dans deux zones séparées par un garnissage et, à l'issue de cette fermentation, le liquide est légèrement agité avant d'être décanté.

Ainsi, le procédé et le dispositif décrits permettent d'obtenir un liquide relativement chargé en alcool (de l'ordre de 8 % en moyenne) avec un temps de séjour total (fermentation et décantation) compatible avec les contraintes industrielles. De plus, la mise en oeuvre de ce procédé et de ce dispositif permet d'obtenir rapidement des levures floculées qui ont une meilleure activité fermentaire, et ce dans des conditions précises et simples à mettre en oeuvre. La simplicité de l'installation est aussi un avantage au niveau industriel pour le procédé et le dispopositif décrits.

L'exemple suivant permettra d'illustrer l'invention.

Le fermenteur utilisé, en acier recouvert d'un revêtement intérieur en peinture polyuréthanne, a une hauteur totale de 2,55 m.

La virole de l'étage de fermentation a une hauteur de 1,70 m et un diamètre intérieur de 0,14 m ; celle de décantation une hauteur de 0,80 m et un diamètre intérieur de 0,20 m. La virole de l'étage de fermentation est munie d'une double enveloppe dans laquelle de l'eau chaude circule.

La paroi conique qui relie ces deux viroles fait avec le plan horizontal un angle de 60° et correspondant à une hauteur dans le plan vertical de 0,05 m.

Le garnissage de l'etage de fermentation est maintenu entre deux grilles situées à 0,9 m et 1,4 m du fond du fermenteur. Sur l'étage de décantation, les grilles sont à 0,4 m et 0,7 m du haut du fermenteur.

Le garnissage est composé d'anneaux en polychlorure de vinyle "FLOCOR R" : diamètre intérieur : 3,5 cm -hauteur : 3,5 cm -surface ondulée à raison de 8 ondes en moyenne sur la hauteur.

Deux piquages sont adaptés sur l'étage de fermentation, l'un juste au-dessous du garnissage et l'autre immédiatement avant le passage dans le déflecteur.

Le substrat à traiter est une mélasse de densité 1,4 et à 235 g/l en saccharose.

Dans un premier temps, on procède à l'ensemencement du fermenteur en injectant en bas du fermenteur une solution préparée en dispersant : 120 g de levure Saccharomyces cerevisiae sèche (levure de boulanger) dans 6 l de mélasse diluée par de l'acide sulfurique dilué (pH : 4,2) contenant 80 g/l de saccharose additionnée de 0,23 g/l d'hydrogénophosphate d'ammonium.

Après que cette solution a été injectée, de la mélasse diluée par de l'acide sulfurique dilué (pH : 4,2) titrant 80 g/l de saccharose est envoyée au fermenteur à raison de 2 l/h, la vitesse ascensionnelle est égale à 0,13 m/h.

Des prélèvements périodiques permettent de suivre l'évolution des cellules. Au bout de 40 h, on observe que pratiquement toutes les levures sont floculées.

A ce moment là, on procède à trois opérations. On abaisse le taux de dilution de la mélasse, de façon que le substrat entrant contienne environ 150 g/l de saccharose. En même temps, on augmente le débit d'alimentation à 3,5 l/h, la vitesse ascensionnelle est alors de 0,23 m/h. L'addition de sels est modifiée : la concentration globale en sels est maintenue à 0,23 g/l mais elle comprend 50 % en poids de sulfate d'ammonium et 50 % en poids d'hydrogénophosphate d'ammonium.

Des prélèvements montrent que 5 à 10 % en poids de la biomasse totale se retrouvent au-dessus du garnissage de l'étage de fermentation et que,en haut de cet étage de fermentation, le liquide alcoolisé contient 7,5 % volumique d'éthanol. Il reste alors environ 10 g/l de saccharose non transformée.

Le procédé utilisant le dispositif décrit permet donc d'obtenir dans des délais convenables à l'échelle industrielle un liquide alcoolisé prêt à être fractionné sans utilisation de centrifugeuses coûteuses. Le temps moyen de séjour du substrat dans l'étage de fermentation est de 4 h dans l'exemple cité et de 2 h dans l'étage de décantation.

## Revendications

Dispositif pour la fermentation alcoolique de liquides sucrés au moyen de levures floculées comportant un étage de fermentation constitué d'un fond et d'un corps de forme cylindrique à axe vertical et un étage de décantation situé au-dessus de l'étage de fermentation constitué d'un corps cylindrique à axe vertical relié tronconiquement au corps cylindrique de l'étage de fermentation et un couvercle fermant ledit étage de décantation, ledit dispositif comportant une tubulure d'amenée des liquides sucrés dans le bas de l'étage de fermentation, une tubulure de départ de jus alcoolisés situé au haut de l'étage de décantation et une tubulure d'évacuation des gaz, ledit dispositif étant caractérisé en ce que :

-chacun des étages de fermentation (1) et de décantation (2) est constitué par une partie inférieure vide (4,5) et une partie supérieure munie d'un garnissage (6,9) maintenu entre deux grilles,

-le diamètre de l'étage de décantation est de 1 à 1,5 fois le diamètre de l'étage de fermentation de façon à ce que l'on puisse réaliser une vitesse ascensionnelle de l'ordre de 0,2 à 0,7 m/h dans l'étage de fermentation et de 0,05 à 0,1 m/h dans l'étage de décantation,

-entre les étages de fermentation et de décantation on aménage au moins un déflecteur (13),

-une tubulure (20) partant du fond de l'étage de fermentation amène le liquide contenant des levures floculées dans une cuve (21) où ledit liquide est additionné d'une quantité convenable d'acide sulfurique pour provoquer la défloculation au moins partielle desdites levures et leur régénération, une tubulure (23) comportant une pompe ramenant les levures ainsi régénérées dans l'étage de fermentation.

Fig.1

Fig.2    Fig.3

Fig. 4a

13
12
28
26
27

Fig. 4b

28
13
30
26
27

Fig.5

29
12

## DOCUMENTS CONSIDERES COMME PERTINENTS

| Categorie | Citation du document avec indication, en cas de besoin, des parties pertinentes | Revendication concernee | CLASSEMENT DE LA DEMANDE (Int. Cl.4) |
|---|---|---|---|
| A | BIOTECHNOLOGY AND BIOENGINEERING, vol. XXVI, no. 7, juillet 1984, pages 742-747, John Wiley & Sons, Inc., New York, US; S.T. JONES et al.: "Ethanol fermentation in a continuous tower fermentor" * Pages 742-744 * | 1 | C 12 M    1/00 |
| A | INTERNATIONAL SUGAR JOURNAL, vol. LXXXII, no. 974, février 1980, pages 44,45, High Wycombe, BUCKS, GB; "Continuous alcohol fermentation in Australia" * Figure * | 1 | |
| A | FR-A-1 307 047  (THE A.P.V. COMPANY) * En entier * | 1 | |
| A | FR-A-2 229 451  (IMPERIAL CHEMICAL INDUSTRIES) | | DOMAINES TECHNIQUES RECHERCHES (Int. Cl 4)  C 12 M   C 12 P |
| D,A | GB-A-2 065 699  (TATE & LYLE) | | |

Le présent rapport de recherche a été etabli pour toutes les revendications

| Lieu de la recherche | Date d'achevement de la recherche | Examinateur |
|---|---|---|
| LA HAYE | 22-07-1986 | NESTBY K. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet anterieur, mais publié à la date de dépôt ou après cette date
D : cité dans la demande
L : cité pour d'autres raisons

& : membre de la même famille, document correspondant

OEB Form 1503 03 82